# EUROPEAN PATENT APPLICATION

(11) **EP 4 606 360 A1**
(43) Date of publication of application: **27.08.2025**
(21) Application number: 23879264.2
(22) Date of filing: 04.10.2023
(51) Int. Cl.: A61F 9/01

(54) **DIGITAL CORNEAL MARKING DEVICE**

(30) Priority: 18.10.2022 ES 202231705 U
(71) Applicant: AJL Ophthalmic, S.A., 01510 Miñano (Alava) (ES)
(72) Inventor: LICARI, José Víctor, 43001 TARRAGONA (ES); CABREJAS CALDINI, Hernán Pablo, 43001 TARRAGONA (ES); BORRÁS SANGENÍS, Ismael, 43001 TARRAGONA (ES)
(74) Representative: Fernandez Pou, Felipe
(86) International application number: PCT/ES2023/070588
(87) International publication number: WO 2024/084115

(57) **Abstract**

The invention relates to a digital corneal marking device formed by a manually handled tool comprising a cylindrical case body (1) that comprises: on the rear part, a motherboard with a control unit (2), battery (3) and a lower cover (4); in the central part, a drive mechanism formed by a guide (5) having a partially cylindrical cross-section on which a motor (6) sits, the motor being coupled to a set of concentric cylindrical ceramic adjustment pieces (7) at each end, wherein the unit is coupled to a movable tip (8) impregnated with non-toxic, non-irritating marking ink; and on the front part, a cylindrical front cover (9) provided with a central through-hole (10) through which the movable tip (8) exits, the front face of the front cover being provided with a series of angular marks (11). The device also comprises an interface (13) formed by a screen and buttons (physical, membrane, tactile or similar), which is arranged on the side of the case body (1) or on the base of the lower cover (4).

## Description

### OBJECT OF THE INVENTION

The present invention relates to a medical device for pre-operative use in cataract and astigmatism operations that provides significant technical advantages over other conventional systems and apparatus.

It proposes a solution for performing ocular marking with an ink-impregnated tip much more precisely than other similar devices on the market, since the corneal marker object of the present invention comprises a motor inside it that rotates until the exact position in degrees required for the orientation of the axis of the toric intraocular lens is obtained.

Until now this process has been done manually and analogically, so the electronic-digital precision offered by the corneal marking device of the present invention considerably improves preparation success, since the toric lenses that are incorrectly positioned per degree have a considerably lower effectiveness.

The industrial application of this invention lies within corneal marking devices and systems, and more specifically, digital corneal marking devices with a precision motor.

### BACKGROUND OF THE INVENTION

Although no invention has been found that is identical to the described invention, the documents found that reflect the state of the art related to same are discussed below.

Thus, document ES2407457 relates to a preoperative toric axis corneal marker, useful for delimiting the correct position of an intraocular lens, an intrastromal ring to be implanted in a patient's eye, the performance of a limbal relaxing incision or any other type of intervention requiring precise marking prior to an intervention on the cornea, such that the marker incorporates fixing means for fixing to a slit lamp and the marker of which comprises a general L-shaped body with a first wing that is anchored to the fixing means for fixing to the slit lamp and a second wing with a hollow cylindrical shape into which there fits a tubular sleeve with at least one small lug in the radial position at its free base, through which the marking will be made on the eye.

Document ES1245260 proposes a portable corneal axis marker to be adapted to smartphones for ophthalmological use, formed by a base adaptable to smartphones, a body and an end for marking ocular tissues, characterised by a base that adapts to the smartphone leaving the camera lens clear, a rigid body having a cylindrical or conical shape and an end for marking ocular tissues that has two elements, an inner and an outer one, that are circular and concentric. The outer element is fixed and has two fixed marks at 0 and 180 degrees and an engraving indicating the axes to be marked. The inner element is movable and rotatable 360 degrees and has two opposing marks. The marks that come into contact with the ocular tissue are blunt and curved, following the anatomy of the cornea and the eyeball.

Document ES2715450 describes a system for vision correction surgery comprising: an ophthalmic detector that can be used to identify a selected location on a cornea of an eye, wherein the selected location is the corneal apex or is along a virtual line connecting the corneal apex and a pupil centre at a given dilation; a marker that can be used to mark the periphery of the cornea with a mark corresponding to the selected location; a corneal suction or flattening device that can be used to deform the cornea; a surgical procedure device that can be used to perform a vision correction surgical procedure at the selected location while the cornea is deformed; and a computer programmed with at least one algorithm to identify the selected location on the undeformed cornea, and an algorithm to re-identify the selected location on the deformed cornea based on the mark, using information provided by the ophthalmic detector, wherein the marker and the surgical procedure device are the same device.

Conclusions: As ensues from the investigation that has been performed, none of documents found solves the contemplated problems like the proposed invention does.

### DESCRIPTION OF THE INVENTION

The digital corneal marking device object of the present invention is formed by a manually handled tool comprising a cylindrical case body that houses a unit with electromechanical elements, oriented to adjust the position in degrees of a movable tip impregnated with non-toxic, non-irritating marking ink, characteristic for interventions of this type.

There are three main sections within the device:
- Located on the rear part is the electronic part, formed by a motherboard with a control unit, battery and, located either on the outer side of the case body or at the base of a lower cover of the device, the interface for controlling the hand-held device through a screen and buttons (physical, membrane, tactile or similar).
- Located in the central part is the drive mechanism, formed by a guide having a partially cylindrical cross-section on which the motor sits, the motor being coupled to a set of concentric cylindrical adjustment parts where the movable tip is coupled. The base where the motor sits has a counterweight and is inserted into two ceramic bearings at each end, where said base works as a pendulum centring the motor on an even level to have the reference between 0 and 180 degrees.
- Located on the front part is a cylindrical front cover provided with a central through-hole through which the movable tip exits, the front face of the front cover being provided with a series of angular marks.

Said cylindrical front cover may be provided with a camera pointing directly at the eye, connected to the electronic circuit of the device, and the image of which is shown on the rear interface screen.

Unless otherwise indicated, all technical and scientific elements used herein have the meaning that is usually understood by a person with skill in the art to which this invention belongs. Methods and materials similar or equivalent to those described in the specification may be used in the practice of the present invention.

Throughout the description and in the claims, the word "comprises" and its variants are not intended to exclude other technical features, additives, components or steps. For those skilled in the art, other objects, advantages and features of the invention may be deduced from both the description and the practical use of the invention.

### BRIEF DESCRIPTION OF THE DRAWINGS

To better understand the present description, several drawings representing a preferred, non-limiting embodiment of the present invention are attached:

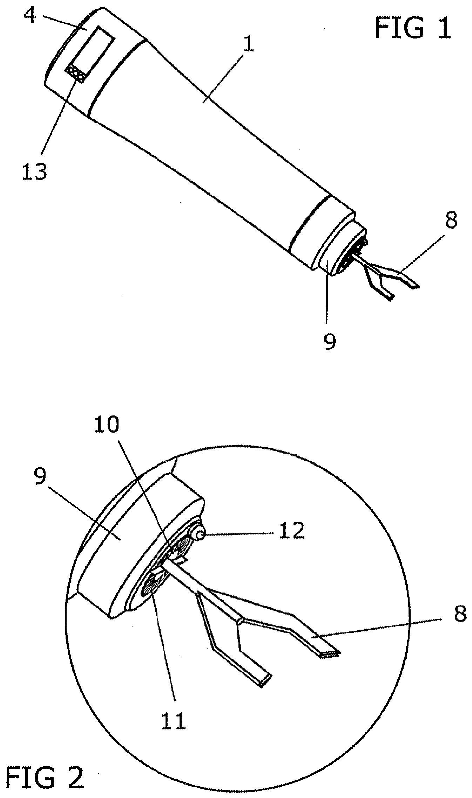

Figure 1: Schematic view of a model of a digital corneal marking device.
Figure 2: Detail view of the cylindrical front cover.

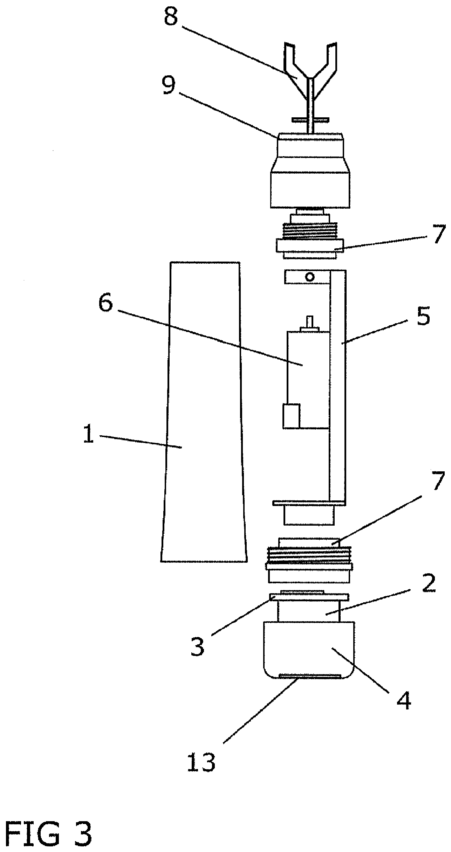

Figure 3: Exploded schematic view of a model of a digital corneal marking device.

The reference numbers appearing in said figures correspond to the following constitutive elements of the invention:
1. Case body
2. Motherboard with a control unit
3. Battery
4. Lower cover
5. Guide
6. Motor
7. Set of concentric cylindrical pieces
8. Movable tip
9. Cylindrical front cover
10. Central through-hole
11. Angular marks
12. Camera
13. Interface (screen + buttons)

### DESCRIPTION OF A PREFERRED EMBODIMENT

A preferred embodiment of the digital corneal marking device object of the present invention, referring to the reference numbers, may be based on a manually handled tool comprising a cylindrical case body (1) that comprises:
- On the rear part, a motherboard with a control unit (2), battery (3) and a lower cover (4).
- In the central part, a drive mechanism, formed by a guide (5) having a partially cylindrical cross-section on which a motor (6) sits, the motor being coupled to a set of concentric cylindrical ceramic adjustment pieces (7) at each end, as a bearing, where said base where the motor (6) sits works as a pendulum centring the motor (6) on an even level to have the reference between 0 and 180 degrees. The unit is coupled to the movable tip (8) impregnated with non-toxic, non-irritating marking ink.
- On the front part, a cylindrical front cover (9) provided with a central through-hole (10) through which the movable tip (8) exits, the front face of the front cover being provided with a series of angular marks (11).

The device is operated from an interface (13) formed by a screen and buttons (physical, membrane, tactile or similar), which is arranged on the side of the case body (1) or on the base of the lower cover (4).

In a different embodiment, the cylindrical front cover (9) is provided with a camera (12) pointing directly at the eye, connected to the electronic circuit of the device, and the image of which is shown on the rear interface screen.

## Claims

1. A digital corneal marking device formed by a manually handled tool comprising a cylindrical case body (1), **characterised in that** it comprises: on the rear part, a motherboard with a control unit (2), battery (3) and a lower cover (4); in the central part, a drive mechanism formed by a guide (5) having a partially cylindrical cross-section on which a motor (6) sits, the motor being coupled to a set of concentric cylindrical ceramic adjustment pieces (7) at each end, wherein the unit is coupled to a movable tip (8) impregnated with non-toxic, non-irritating marking ink; and on the front part, a cylindrical front cover (9) provided with a central through-hole (10) through which the movable tip (8) exits, the front face of the front cover being provided with a series of angular marks (11); and the device also comprises an interface (13) formed by a screen and buttons (physical, membrane, tactile or similar), which is arranged on the side of the case body (1) or on the base of the lower cover (4).

2. The digital corneal marking device according to claim 1, wherein the cylindrical front cover (9) is provided with a camera (12) pointing directly at the eye, connected to the electronic circuit of the device, and the image of which is shown on the rear interface screen.
